**Europäisches Patentamt**

**European Patent Office**

**Office européen des brevets**

(19)

(11) Veröffentlichungsnummer: **0 150 821**
**B1**

(12) # EUROPÄISCHE PATENTSCHRIFT

(45) Veröffentlichungstag der Patentschrift:
**01.04.87**

(21) Anmeldenummer: **85100742.7**

(22) Anmeldetag: **25.01.85**

(51) Int. Cl.⁴: **C 07 C 179/053**, C 07 C 178/00,
C 07 C 45/33, C 07 C 49/403,
C 07 C 29/50, C 07 C 35/08

(54) **Verfahren zur kontinuierlichen Herstellung von Cyclohexylhydroperoxid sowie Cyclohexanol und Cyclohexanon enthaltenden Gemischen.**

(30) Priorität: **01.02.84 DE 3403410**

(43) Veröffentlichungstag der Anmeldung:
**07.08.85 Patentblatt 85/32**

(45) Bekanntmachung des Hinweises auf die Patenterteilung:
**01.04.87 Patentblatt 87/14**

(84) Benannte Vertragsstaaten:
**BE CH DE FR GB IT LI NL**

(56) Entgegenhaltungen:
**EP - A - 0 031 113**
**GB - A - 1 085 892**
**GB - A - 1 160 457**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,
Carl-Bosch-Strasse 38, D-6700 Ludwigshafen (DE)**

(72) Erfinder: **Brunner, Erwin, Dr., Neuhausener Weg 1,
D-6700 Ludwigshafen (DE)**
Erfinder: **Grosskinsky, Otto-Alfred, Dr.,
Semmelweisstrasse 8, D-6700 Ludwigshafen (DE)**
Erfinder: **Herrmann, Guenter, Dr., Haeuserstrasse 21,
D-6900 Heidelberg (DE)**
Erfinder: **Krokoszinski, Roland, Dr., Im Eiertal 4a,
D-6719 Weisenheim (DE)**
Erfinder: **Magnussen, Peter, Dr.,
Professor-Dillinger-Weg 25, D-6702 Bad Duerkheim (DE)**

ACTORUM AG

**Beschreibung**

In der DE-OS 2 951 956 wird ein Verfahren beschrieben zur Herstellung von Gemischen aus Cyclohexylhydroperoxid, Cyclohexanol und Cyclohexanon mit einem hohen Gehalt an Peroxiden, bei dem man die Umsetzung von Cyclohexan mit molekularem Sauerstoff in flüssiger Phase in mehreren, hintereinander geschalteten Stufen durchführt und von Stufe zu Stufe die Temperatur um jeweils mindestens 5°C senkt und in jede Stufe molekularen Sauerstoff einleitet. Die hierbei erzielten Ausbeuten an Cyclohexylhydroperoxid sind noch verbesserungsbedürftig. Aus der DE-OS 1 593 700 und der GB-PS 1 085 892 sind auch bereits Verfahren bekannt, bei denen man zunächst molekularen Sauerstoff in Cyclohexan bei einer tieferen Temperatur löst und das gelösten molekularen Sauerstoff enthaltende Cyclohexan in einer zweiten Stufe bei höherer Temperatur durch eine rohrförmige Reaktionszone leitet. Entsprechend den dort beschriebenen Arbeitsweisen erhält man jedoch vorwiegend Cyclohexanol und Cyclohexanon sowie erhebliche Mengen an Säuren, jedoch nur untergeordnete Menge an Cyclohexylhydroperoxid. Die Säuren sind unerwünschte Nebenprodukte, die durch die weitere Oxidation von Cyclohexanon mit Sauerstoff entstehen. Um eine möglichst hohe Ausbeute an Wertprodukten zu erhalten und den Anteil an Nebenprodukten zu minimieren, ist es notwendig, die Reaktion so zu führen, dass sie bei dem zuerst gebildeten Cyclohexylhydroperoxid stehen bleibt bzw. dieses zum Hauptprodukt wird. Das Cyclohexylhydroperoxid kann dann in einer weiteren Stufe in Abwesenheit von molekularem Sauerstoff mit praktisch quantitativer Ausbeute zu Cyclohexanol und Cyclohexanon umgesetzt werden; durch Umsetzung in Gegenwart von Olefinen können dabei mit geeigneten Katalysatoren auch Epoxide hergestellt werden.

Es war deshalb die technische Aufgabe gestellt, bei der Oxidation von Cyclohexan mit molekularem Sauerstoff die Reaktion so zu führen, dass im wesentlichen Cyclohexylhydroperoxid erhalten wird und der Gehalt an Cyclohexanon und Carbonsäuren möglichst niedrig gehalten wird.

Diese Aufgabe wird gelöst in einem Verfahren zur kontinuierlichen Herstellung von Cyclohexylhydroperoxid sowie Cyclohexanol und Cyclohexanon enthaltenden Gemischen mit einem hohen Gehalt an Cyclohexylhydroperoxid durch Oxidation von Cyclohexan mit molekularem Sauerstoff oder solches enthaltenden Gasen in flüssiger Phase bei dem man

1. in einer 1. Stufe molekularen Sauerstoff in Cyclohexan durch in Berührung bringen mit molekularem Sauerstoff oder solchen enthaltenden Gasen bei einer Temperatur von 10 bis 110°C unter erhöhtem Druck löst und

2. in einer 2. Stufe das gelösten molekularen Sauerstoff enthaltende Cyclohexan bei einer Temperatur von 130 bis 250°C und unter erhöhtem Druck durch eine rohrförmige Reaktionszone leitet,

dadurch gekennzeichnet, dass man das gelösten molekularen Sauerstoff enthaltende Cyclohexan angenähert in Pfropfenströmung ohne Ausbildung einer Gasphase durch eine langgestreckte rohrförmige Reaktionszone leitet, wobei die Verweilzeit so bemessen ist, dass sie zwischen

a) der Zeit, zu der 50% der Menge an gelöstem molekularem Sauerstoff verbraucht ist und

b) dem 1,2fachen der Zeit die zum Verbrauch von 99,9% des gelösten Sauerstoffs erforderlich ist

liegt.

Das neue Verfahren hat den Vorteil, dass man höhere Ausbeuten an Cyclohexylhydroperoxid als bisher erzielt und der Gehalt an Cyclohexanon und Carbonsäuren niedrig gehalten wird.

Zunächst wird in einer 1. Stufe molekularer Sauerstoff in Cyclohexan gelöst. Hierbei bringt man Cyclohexan mit molekularem Sauerstoff oder solche enthaltenden Gasen in Berührung bringt. In der Regel geht man von Gasen mit einem Gehalt an molekularem Sauerstoff von mehr als 20%, vorteilhaft über 90%, insbesondere über 99% aus. Die restlichen Anteile sind Inertgase, wie Stickstoff, Kohlendioxid und Edelgase. Hierbei hält man eine Temperatur von 10 bis 110°C, insbesondere 15 bis 80°C, ein. Das Lösen des molekularen Sauerstoffs in Cyclohexan wird unter erhöhtem Druck, vorteilhaft unter einem Druck von 10 bis 200 bar, insbesondere 15 bis 60 bar, durchgeführt.

In der Regel hält man einen Sättigungsgrad an molekularem Sauerstoff von 60 bis 100% ein.

Es ist möglich, Cyclohexan unter den jeweils angewandten Temperatur- und Druckbedingungen vollständig mit molekularem Sauerstoff zu sättigen. Aus Sicherheitsgründen ist es jedoch angezeigt, einen Sättigungsgrad bei den jeweils angewandten Temperatur- und Druckbedingungen bis 90%, z.B. von 60 bis 90%, einzuhalten.

Zweckmässig führt man das Sättigen von Cyclohexan mit molekularem Sauerstoff in Vorrichtungen, wie sie hierfür in der Technik für das Lösen von Gasen in Flüssigkeiten gebräuchlich sind, z.B. Füllkörperkolonnen oder Rührkesseln, wobei molekularer Sauerstoff in fein verteilter Form eingeleitet wird, durch.

Das gelösten molekularen Sauerstoff enthaltende Cyclohexan wird dann in einer 2. Stufe durch eine langgestreckte rohrförmige Reaktionszone geleitet. Hierbei hält man angenähert Pfropfenströmung ein, d.h. es ist darauf zu achten, dass keine oder nur sehr geringe Rückvermischung stattfindet. Dies wird z.B. erzielt durch langgestreckte Reaktionszonen mit einem Verhältnis von Länge: Durchmesser von 100:1 bis 1000:1 oder durch langestreckte röhrenförmige Reaktionszonen mit Einbauten, wie z.B. Raschigringen, mit einem Verhältnis von Länge: Durchmesser von 10:1 bis 100:1.

In der 2. Stufe hält man eine Temperatur von 130 bis 250°C ein. Besonders bewährt hat sich eine Temperatur von 150 bis 200°C. Vorzugsweise senkt man die Reaktionstemperatur im Verlauf der rohrförmigen Reaktionszone ab. Dies erfolgt z.B. kontinuierlich oder stufenweise, z.B. in 3 bis 6 Stufen, wobei man gegenüber der Temperatur am Eingang der rohrförmigen Reaktionszone eine Absenkung der Temperatur bis zum Ausgang der rohrförmigen Reaktionszone, vorteilhaft von 10 bis 40°C, insbesondere 15 bis 30°C, einhält. Es versteht sich, dass durch das Absenken der Temperatur die untere Grenze der Reaktionstemperatur, wie sie oben angegeben ist, nicht unterschritten wird.

Vorteilhaft ist die langgestreckte Reaktionszone als Rohrschlange ausgebildet. Es ist vorteilhaft mehrere Rohrschlangen, z.B. bis zu 5, vorzugsweise mit abnehmender Länge hintereinander zu schalten. Hierbei hat es sich bewährt, die Temperatur von Abschnitt zu Abschnitt zu senken. Eine andere vorteilhafte Ausführung der langgestreckten Reaktionszone besteht aus Röhrenbündeln mit parallelen Rohren. Vorzugsweise schaltet man mehrere Röhrenbündel, z.B. bis zu 5, insbesondere mit abnehmender Länge hintereinander, wobei es sich bewährt hat, die Temperatur von Röhrenbündel zu Röhrenbündel zu senken.

Der in der 2. Stufe angewandte Druck ist zweckmässig gleich oder grösser als der Sättigungsdruck. Es wird darauf geachtet, dass bei der jeweils angewandten Temperatur der Druck so hoch ist, dass sich keine Gasphase ausbildet. In der Regel hält man Drücke von 40 bis 100 bar ein. Vorteilhaft liegt der Druck in der 2. Stufe mindestens um 10 bar höher als der Sättigungsdruck.

Die Verweilzeit in der 2. Stufe ist so bemessen, dass sie zwischen

a) der Zeit, zu der 50% der Menge an gelöstem molekularem Sauerstoff verbraucht ist und

b) dem 1,2fachen der Zeit, die zum Verbrauch von 99,9% des gelösten Sauerstoffs erforderlich ist,

liegt.

Vorteilhaft hält man eine Verweilzeit ein, die zwischen der Zeit, zu der 80% der Menge an gelöstem molekularem Sauerstoff verbraucht ist und der Zeit, die zum Verbrauch von 99,9% des gelösten molekularen Sauerstoffs erforderlich ist, liegt.

Vorteilhaft hält man in der 2. Zone einen Umsatz von 1 bis 8 Gew.-%, insbesondere 2 bis 6 Gew.-%, bezogen auf Cyclohexan, ein.

Es hat sich auch bewährt wenn man im Verlauf der 2. Stufe an mindestens einer weiteren Stelle, z.B. 1 bis 4 Stellen, die vorteilhaft in der ersten Hälfte der Stufe 2 angeordnet sind, molekularen Sauerstoff enthaltendes Cyclohexan zuführt.

Um die Induktionsperiode in der 2. Stufe zu verkürzen, setzt man vorteilhaft Starter, z.B. Peroxide, insbesondere Cyclohexylhydroperoxid oder solches enthaltendes Reaktionsgemisch zu, z.B. in einer Menge von 0,1 bis 0,5 Gew.-%, bezogen auf Cyclohexan.

Die mit dem Reaktionsgemisch in Berührung kommenden Flächen, insbesondere in der 2. Stufe, sind vorteilhaft emailliert oder passiviert. Die Passivierung erreicht man z.B. durch Behandeln mit Pyrophosphaten. Geeignete Pyrophosphate sind z.B. Na-Pyrophosphat und K-Pyrophosphat. Besonders bewährt hat es sich, wenn man dem Cyclohexan geringe Mengen z.B. bis zu 10 ppm Pyrophosphat fortwährend oder in Intervallen zusetzt.

Das erhaltene Reaktionsgemisch enthält neben nichtumgesetztem Cyclohexan Cyclohexylhydroperoxid, Cyclohexanol, untergeordnete Mengen an Cyclohexanon und geringe Mengen an anderen sauerstoffhaltigen Oxidationsprodukten, wie Ester und Carbonsäuren.

Aus dem erhaltenen Reaktionsgemisch werden vor der weiteren Verarbeitung vorteilhaft ein Teil der sauren Bestandteile durch Wasser ausgewaschen. Die weitere Umsetzung des Cyclohexylhydroperoxids zu Cyclohexanol und Cyclohexanon unter Mitverwendung von katalytisch aktiven Metallverbindungen erfolgt beispielsweise wie in der DE-OS 2 352 378 beschrieben. Cyclohexanon und Cyclohexanol dienen zur Herstellung von Adipinsäure und Caprolactam.

Das Verfahren nach der Erfindung sei an folgenden Beispielen veranschaulicht.

*Beispiel 1*

Einem Hochdruckgefäss mit Rührer und einem Volumen von 1000 cm$^3$ aus nichtrostendem Stahl werden stündlich 101 g Cyclohexan zugeführt und bei 25°C und unter einem Druck von 15 bar molekularer Sauerstoff mit einem Gehalt an Sauerstoff von 100 Vol.% in feiner Verteilung eingeleitet. Das so mit molekularem Sauerstoff gesättigte Cyclohexan wird in einer Menge von 101 g pro Stunde in ein Glasrohr von 0,5 cm Innendurchmesser und 5 m Länge geleitet. Das Glasrohr wurde über die gesamte Länge auf eine Temperatur von 170°C erhitzt. Um die Bildung von Gasen zu vermeiden, wurde im Glasrohr ein Druck von etwa 20 bar aufrechterhalten. Aus der Menge des zugeleiteten Cyclohexans ergibt sich eine Verweilzeit von 36 Minuten, die dem 1,0fachen der Zeit, die zum Verbrauch von 99,9% des gelösten molekularen Sauerstoffs erforderlich ist, entspricht. Das erhaltene Reaktionsgemisch enthält neben Cyclohexan 1,98 Gew.-% Cyclohexylhydroperoxid, 0,23 Gew.-% Cyclohexanol, 0,095 Gew.-% Cyclohexanon sowie 0,08 Gew.-% Carbonsäuren. Die Selektivität des umgesetzten Cyclohexans zu Cyclohexylhydroperoxid betrug 81,3%, dienige zu Cyclohexanon 4,65% und diejenige zu Cyclohexanol 10,8%. Die Selektivität an umgesetztem Cyclohexan zu Cyclohexylhydroperoxid, Cyclohexanol und Cyclohexanon insgesamt betrug 96,7%. Der Anteil an Cyclohexylhydroperoxid an den letztgenannten drei Produkten betrug 0,841.

*Beispiel 2*

Man verfährt wie in Beispiel 1 beschrieben, erhitzt das Glasrohr (Stufe 2) auf eine Temperatur von 180°C, beschickt es stündlich mit 198,3 g Cyclohexan, das mit molekularem Sauerstoff gesättigt ist. Es ergibt sich eine Verweilzeit von 18 Minuten. Dies entspricht einer Zeit, in der 99,9% des gelösten molekularen Sauerstoffs verbraucht sind. Das erhaltene Reaktionsgemisch enthält neben Cyclohexan 1,89 Gew.-% Cyclohexylhydroperoxid, 0,30 Gew.-% Cyclohexanol, 0,11 Gew.-% Cyclohexanon sowie 0,05 Gew.-% Carbonsäuren. Die Selektivität des umgesetzten Cyclohexans zum Cyclohexylhydroperoxid betrug 69,0%, diejenige zu Cyclohexanon 6,4% und diejenige zu Cyclohexanol 20,3%. Die Selektivität des umgesetzten Cyclohexans zu Cyclohexylhydroperoxid, Cyclohexanon und Cyclohexanol betrug insgesamt 95,6%. Der Anteil an Cyclohexylhydroperoxid an den letztgenannten drei Wertprodukten betrug 0,800.

*Beispiel 3*

Man verfährt wie in Beispiel 1 beschrieben, erhitzt das Glasrohr (Stufe 2) auf 170° und hält einen Druck von 20 bar ein. Stündlich führt man 114 g Cyclohexan, das mit molekularem Sauerstoff gesättigt ist, zu. Bei einer Verweilzeit von 32 Minuten wurden 56% des gelösten Sauerstoffs umgesetzt. Das erhaltene Reaktionsgemisch enthielt neben Cyclohexan 1,6 Gew.-% Cyclohexylhydroperoxid, 0,12 Gew.-% Cyclohexanol, 0,05 Gew.-% Cyclohexanon sowie 0,04 Gew.-% Carbonsäuren. Die Selektivität, bezogen auf umgesetztes Cyclohexan zu Cyclohexylhydroperoxid betrug 87,2%, diejenige zu Cyclohexanol 6,8% und diejenige zu Cyclohexanon 3,5%. Die Selektivität, bezogen auf umgesetztes Cyclohexan zu Cyclohexylhydroperoxid, Cyclohexanol und Cyclohexanon betrug 97,5%. Der Anteil an Cyclohexylhydroperoxid an den letzgenannten drei Produkten betrug 0,890.

*Vergleichsbeispiel 1*

Man verfährt wie in Beispiel 1 beschrieben, ersetzt das Glasrohr (Stufe 2) jedoch durch einen Behälter mit Rührer von 100 cm$^3$ Inhalt und führt dem Rührbehälter stündlich 116 g mit molekularem Sauerstoff gesättigtes Cyclohexan zu und hält eine Temperatur von 170°C, einen Druck von 20 bar und eine mittlere Verweilzeit von 36 Minuten ein. Das erhaltene Reaktionsgemisch hat einen relativen Anteil an Cyclohexylhydroperoxid von 0,47, bezogen auf das gebildete Cyclohexylhydroperoxid, Cyclohexanol und Cyclohexanon. Die Selektivität, bezogen auf umgesetztes Cyclohexan betrug 91%.

*Vergleichsbeispiel 2*

Man verfährt wie in Vergleichsbeispiel 1 beschrieben und hält in dem gerührten Behälter (Stufe 2) eine Temperatur von 180°C, einen Druck von 25 bar und eine Verweilzeit von 18 Minuten ein. Das erhaltene Reaktionsgemisch hatte einen relativen Anteil an Cyclohexylhydroperoxid von 0,50, bezogen auf den Gehalt an Cyclohexylhydroperoxid, Cyclohexanol und Cyclohexanon. Die Selektivität, bezogen auf umgesetztes Cyclohexan betrug für Cyclohexylhydroperoxid 44,4%, für Cyclohexanon 10,3% und für Cyclohexanol 34,8%. Hierauf ergibt sich eine Gesamtselektivität für die letztgenannten drei Verbindungen von 89,5%.

**Patentansprüche**

1. Verfahren zur kontinuierlichen Herstellung von Cyclohexylhydroperoxid sowie Cyclohexanol und Cyclohexanon enthaltenden Gemischen mit einem hohen Gehalt an Cyclohexylhydroperoxid durch Oxidation von Cyclohexan mit molekularem Sauerstoff oder solches enthaltenden Gasen in flüssiger Phase bei dem man

1. in einer 1. Stufe molekularen Sauerstoff in Cyclohexan durch in Berührung bringen mit molekularem Sauerstoff oder solches enthaltenden Gasen bei einer Temperatur von 10 bis 110°C unter erhöhtem Druck löst und

2. in einer 2. Stufe das gelösten molekularen Sauerstoff enthaltende Cyclohexan bei einer Temperatur von 130 bis 250°C und unter erhöhtem Druck durch eine rohrförmige Reaktionszone leitet,

dadurch gekennzeichnet, dass man das gelösten molekularen Sauerstoff enthaltende Cyclohexan angenähert in Pfropfenströmung ohne Ausbildung einer Gasphase durch eine langgestreckte, rohrförmige Reaktionszone leitet, wobei die Verweilzeit so bemessen ist, das sie zwischen

a) der Zeit, in der 50% der Menge an gelöstem, molekularem Sauerstoff verbraucht ist und

b) dem 1,2fachen der Zeit, die zum Verbrauch von 99,9% des gelösten molekularen Sauerstoffs erforderlich ist,

liegt.

2. Verfahren nach Anspruch 1, dadurch gekennzeichnet, dass man in der 1. Stufe einen Sättigungsgrad an molekularem Sauerstoff von 60 bis 100% einhält.

3. Verfahren nach den Ansprüchen 1 und 2, dadurch gekennzeichnet, dass man in der 2. Stufe einen Druck einhält, der mindestens 10 bar über dem Sättigungsdruck liegt.

4. Verfahren nach den Ansprüchen 1 bis 3, dadurch gekennzeichnet, dass man in der 2. Stufe eine Verweilzeit einhält, die erforderlich ist, um 80 bis 99,9% des gelösten molekularen Sauerstoffs zu verbrauchen.

5. Verfahren nach den Ansprüchen 1 bis 4, dadurch gekennzeichnet, dass die röhrenförmige Reaktionszone in Stufe 2 ein Verhältnis von Länge zu Durchmesser von 100:1 bis 1000:1 aufweist.

6. Verfahren nach den Ansprüchen 1 bis 5, dadurch gekennzeichnet, dass man in der 2. Stufe die Temperatur im Verlauf der rohrförmigen Reaktionszone absenkt.

7. Verfahren nach den Ansprüchen 1 bis 6, dadurch gekennzeichnet, dass man in der 2. Stufe die Temperatur im Verlauf der rohrförmigen Reaktionszone um 10 bis 40°C absenkt.

8. Verfahren nach den Ansprüchen 1 bis 7, dadurch gekennzeichnet, dass man im Verlauf der zweiten Stufe an mindestens einer weiteren Stelle molekularen Sauerstoff enthaltendes Cyclohexan zuführt.

9. Verfahren nach den Ansprüchen 1 bis 8, dadurch gekennzeichnet, dass man dem verwendeten Cyclohexan Alkalipyrrophosphate zusetzt.

10. Verfahren nach den Ansprüchen 1 bis 9, dadurch gekennzeichnet, dass man dem in die 2. Stufe eintretenden Reaktionsgemisch Peroxide zusetzt.

11. Verfahren nach den Ansprüchen 1 bis 10, dadurch gekennzeichnet, dass man als Starter Cyclohexylhydroperoxid verwendet.

**Claims**

1. A process for the continuous preparation of a mixture containing cyclohexyl hydroperoxide, cyclohexanol and cyclohexanone and having a high cyclohexyl hydroperoxide content, by oxidation of cyclohexane with molecular oxygen, or a gas containing this, in the liquid phase, in which

1. in a first stage, molecular oxygen is dissolved in cyclohexane by bringing the latter into contact with molecular oxygen, or with a gas containing this, at from 10 to 110°C and under superatmospheric pressure, and

2. in a second stage, the cyclohexane containing dissolved molecular oxygen is passed through a tubular reaction zone at from 130 to 250°C and under superatmospheric pressure,

wherein the cyclohexane containing dissolved molecular oxygen is passed through an elongated tubular reaction zone substantially in plug flow, without the formation of a gas phase, the residence time being the period between

a) the point in time at which 50% of the amount of dissolved molecular oxygen has been consumed, and

b) the end of the period corresponding to 1.2 times the time required for the consumption of 99.9% of the dissolved molecular oxygen.

2. A process as claimed in claim 1, wherein, in the first stage, the degree of saturation with molecular oxygen is maintained at from 60 to 100%.

3. A process as claimed in claims 1 and 2, wherein, in the second stage, the pressure maintained is at least 10 bar above the saturation pressure.

4. A process as claimed in claims 1 to 3, wherein, in the second stage, the residence time maintained is the time required to consume from 80 to 99.9% of the dissolved molecular oxygen.

5. A process as claimed in claims 1 to 4, wherein the tubular reaction zone in the second stage has a length/diameter ratio of from 100:1 to 1000:1.

6. A process as claimed in claims 1 to 5, wherein, in the second stage, the temperature is reduced along the tubular reaction zone.

7. A process as claimed in claims 1 to 6, wherein, in the second stage, the temperature is reduced by 10-40°C along the tubular reaction zone.

8. A process as claimed in claims 1 to 7, wherein, during the second stage, cyclohexane containing molecular oxygen is fed in at at least one further point.

9. A process as claimed in claim 1 to 8, wherein an alkali metal pyrophosphate is added to the cyclohexane used.

10. A process as claimed in claim 1 to 9, wherein a peroxide is added to the reaction mixture entering the second stage.

11. A process as claimed in claims 1 to 10, wherein cyclohexyl hydroperoxide is used as initiator.

**Revendications**

1. Procédé de préparation continue de mélanges contenant de l'hydroperoxyde de cyclohexyle ainsi que du cyclohexanol et de la cyclohexanone, à teneur élevée en hydroperoxyde de cyclohexyle, per l'oxydation du cyclohexane à l'aide d'oxygène moléculaire ou de gaz qui contiennent ce dernier, en phase liquide, suivant lequel

1. dans une première étape, on dissout de l'oxygène moléculaire dans du cyclohexane par mise en contact avec de l'oxygène moléculaire ou de gaz contenant ce dernier, à une température de 10 à 110°C, sous pression élevée et

2. dans une seconde étape, on fait passer le cyclohexane contenant de l'oxygène moléculaire dissous, à une température de 130 à 250°C et sous pression élevée, à travers une zone réactionnelle tubulaire,

caractérisé en ce qu'on fait passer le cyclohexane contenant de l'oxygène moléculaire dissous approximativement suivant un écoulement tampon sans formation de phase gazeuse, à travers une zone réactionnelle tubulaire allongée, où on règle la durée de séjour de façon à ce qu'elle soit comprise entre

a) la durée en laquelle 50% de la quantité d'oxygène moléculaire dissous sont consommés et

b) 1,2 fois la durée qui est nécessaire à la consommation de 99,9% de l'oxygène moléculaire dissous.

2. Procédé suivant la revendication 1, caractérisé en ce que l'on maintient un degré de saturation en oxygène moléculaire de 60 à 100% au cours de la première étape.

3. Procédé suivant les revendications 1 et 2, caractérisé en ce que l'on maintient une pression au cours de la seconde étape, qui est supérieure d'au moins 10 bars à la pression de saturation.

4. Procédé suivant les revendications 1 à 3, caractérisé en ce que l'on impose, dans la seconde étape, une durée de séjour qui est nécessaire à la consommation de 80 à 99,9% de l'oxygène moléculaire dissous.

5. Procédé suivant les revendications 1 à 4, caractérisé en ce que la zone réactionnelle tubulaire présente un rapport de sa longueur à son diamètre de 100:1 à 1000:1 dans la seconde étape.

6. Procédé suivant les revendications 1 à 5, caractérisé en ce que l'on réduit la température tout au long de la zone réactionnelle tubulaire dans la seconde étape.

7. Procédé suivant les revendications 1 à 6, caractérisé en ce que l'on réduit la température tout au long de la zone réactionnelle tubulaire de 10 à 40°C au cours de la seconde étape.

8. Procédé suivant les revendications 1 à 7, caractérisé en ce que l'on introduit du cyclohexane contenant de l'oxygène moléculaire en au moins un autre endroit au cours du déroulement de la seconde étape.

9. Procédé suivant les revendications 1 à 8, caractérisé en ce que l'on ajoute des pyrophosphates d'alcalis au cyclohexane utilisé.

10. Procédé suivant les revendications 1 à 9, caractérisé en ce que l'on ajoute des peroxydes au mélange réactionnel qui pénètre dans la seconde étape.

11. Procédé suivant les revendications 1 à 10, caractérisé en ce que l'on utilise l'hydroperoxyde de cyclohexyle à titre d'amorceur.